# EUROPÄISCHE PATENTSCHRIFT

(11) **EP 1 301 610 B1**
(45) Veröffentlichungstag und Bekanntmachung des Hinweises auf die Patenterteilung: **05.04.2006**
(21) Anmeldenummer: 01967185.8
(22) Anmeldetag: 16.07.2001
(51) Int. Cl.: C12N 15/82, C12N 15/29, C07K 14/415, A01H 1/06, A01H 5/00, A01H 5/10, C12N 15/56

(54) **VERFAHREN ZUR BEEINFLUSSUNG DES SINAPINGEHALTS IN TRANSGENEN PFLANZENZELLEN UND PFLANZEN**
METHOD FOR INFLUENCING THE CONTENT OF SINAPINE IN TRANSGENIC PLANT CELLS AND PLANTS
PROCEDE PERMETTANT D'INFLUENCER LA TENEUR EN SINAPINE DE PLANTES ET CELLULES VEGETALES TRANSGENIQUES

(30) Priorität: 14.07.2000 DE 10034320
(43) Veröffentlichungstag der Anmeldung: 16.04.2003
(73) Patentinhaber: Institut für Pflanzenbiochemie, 06120 Halle/Saale (DE)
(72) Erfinder: MILKOWSKI, Carsten, 06114 Halle (Saale) (DE); BAUMERT, Alfred, 06108 Halle (Saale) (DE); STRACK, Dieter, 38300 Wolfenbüttel (DE)
(74) Vertreter: Neuefeind, Regina
(86) Internationale Anmeldenummer: PCT/EP2001/008199
(87) Internationale Veröffentlichungsnummer: WO 2002/006320

(56) Entgegenhaltungen:
- DATABASE EMBL [Online] 1. Januar 1998 (1998-01-01) Database accession no. O23401 XP002201199
- MILKOWSKI M., ET AL.: "Cloning and heterologous expression of a rape cDNA encoding UDP-glucose:sinapate glucosyltransferase" PLANTA, Bd. 211, 2000, Seiten 883-886, XP002201197
- DATABASE EMBL [Online] 11. August 2000 (2000-08-11) Database accession no. AF287143 XP002201200
- LIM ENG-KIAT ET AL: "Identification of glucosyltransferase genes involved in sinapate metabolism and lignin synthesis in Arabidopsis" JOURNAL OF BIOLOGICAL CHEMISTRY, AMERICAN SOCIETY OF BIOLOGICAL CHEMISTS, BALTIMORE, MD, US, Bd. 276, Nr. 6, 9. Februar 2001 (2001-02-09), Seiten 4344-4349, XP002172233 ISSN: 0021-9258
- SCHAWN X., ET AL.: "enzymology of UDP-glucose:sinapic acid glucosyltransferasefrom brassica napus" PHYTOCHEMISTRY, Bd. 49, Nr. 2, 1998, Seiten 307-318, XP004290102

## Beschreibung

Die Erfindung betrifft ein Verfahren zur Beeinflussung des Sinapingehalts in transgenen Pflanzenzellen und Pflanzen. Insbesondere betrifft die Erfindung die Inhibierung der enzymatischen Aktivität einer UDP-Glucose:Sinapinsäure-Glucosyltransferase (SGT) in transgenen Pflanzenzellen. Des weiteren betrifft die Erfindung Nukleinsäuremoleküle, die eine DNA-Sequenz enthalten, die ein Protein mit der enzymatischen Aktivität einer SGT kodiert. Ferner betrifft die Erfindung transgene Pflanzen und Pflanzenzellen, die ein erfindungsgemäßes Nukleinsäuremolekül enthalten und aufgrund dessen im Vergleich zu Wildtyppflanzen bzw. -zellen einen verringerten Gehalt an Sinapin aufweisen, sowie Ernteprodukte und Vermehrungsmaterial der transgenen Pflanzen.

In den letzten 15 Jahren hat sich die Produktion pflanzlicher Öle weltweit stark erhöht. Der Anteil an Rapsöl wuchs dabei stärker als der anderer Ölsorten. Raps wird hauptsächlich zur Gewinnung hochwertiger Öle für die Lebensmittelindustrie angebaut. Das gewonnene Rapsöl wird aber auch zunehmend in anderen Industriezweigen eingesetzt. Außerdem ist die Verwendung von Rapsölen als nachwachsender Rohstoff zum Ersatz der begrenzten Ressourcen an fossiler Energie von wachsendem Interesse.

Als Rückstand der Ölgewinnung fallen große Mengen Rapsschrot bzw. Rapsmehl an, das als wertvolles proteinreiches Futtermittel Verwendung findet. So wird es in begrenztem Umfang in der Tierernährung eingesetzt. Rapsmehl hat einen mit Sojamehl vergleichbaren Proteingehalt von etwa 20% im Samen. Auch der Anteil an essentiellen Aminosäuren entspricht etwa dem des Sojamehls, so dass Rapsprotein ernährungsphysiologisch mit dem Sojaprotein vergleichbar ist. Es besteht somit ein großes Interesse, das Rapsprotein auch für die menschliche Ernährung zugänglich zu machen.

Der Einsatz von Rapsmehl in der Tierernährung und die Verwendung von Rapsproteinen in der menschlichen Ernährung ist jedoch durch einen hohen Anteil an antinutritiven Komponenten eingeschränkt. Glucosinolate (D.W. Griffiths, J. Hortic (1998) Sci. & Biotechnol. 73: 1-18), die zunächst als wichtigste limitierende antinutritive Faktoren angesehen wurden, sind durch die Einführung von neuen Rapssorten, wie beispielsweise Canola, weitgehend reduziert worden und können durch züchterische Bearbeitung ganz eliminiert werden.

Eine andere wichtige Stoffgruppe mit antinutritiven Eigenschaften sind phenolische Inhaltsstoffe (A. Bouchereau et al. (1991), Phytochemistry 30:1873-1881). Der Gehalt an Phenolen in Rapsmehl liegt etwa dreißigmal so hoch wie in Sojamehl. Phenolische Verbindungen sind an der dunklen Farbe, dem bitteren Geschmack und einem unangenehmen Geruch sowie der adstringierenden Eigenschaften des Rapsmehls beteiligt (R. Blair und D. R Reichert (1984), J. Sci. Food Agric. 35: 29-35). Lösliche phenolische Verbindungen werden im Rapssamen hauptsächlich in den Kotyledonen der Embryonen akkumuliert. Herausragende Komponente ist das Sinapoylcholin (Sinapin), dessen Gehalt im Rapsmehl bei 1 bis 2 % liegt.

Sinapin wird auch in Verbindung gebracht mit dem Auftreten von Fischgeruch in Eiern von Legehennen nach der Fütterung mit Rapssamen. Dieser Geruch ist auf die Akkumulation von Triethylamin in den Eiern zurückzufdhren (R. G. Fenwick et al. (1984), J. Sci. Food Agric. 35: 757-761). Es wird postuliert, dass Rapssamen-Tannine die Triethylaminoxidase in Hühnern durch Komplexbildung hemmen und die Bildung des geruchlosen Triethylaminoxids verhindern.

Wie bereits vorstehend erwähnt, ist ein typisches Merkmal vieler Brassicaceen (*Cruciferae*) die Akkumulation von phenolischen Cholinestern in den Kotyledonen der Samen-Embryonen. Sinapoylcholin (Sinapin) ist mit 1 bis 2 % der Trockenmasse der Samen der herausragende Vertreter dieser Stoffklasse (K. Krygier et al. (1982), J. Food Chem. 30: 661-663); A. Boucherau et al., vide supra). Andere Hydroxyzimtsäureester sind nur in geringen Mengen vorhanden (T. W. Fenton et al. (1980), J. Food Sci. 45: 1703-1705). Die biochemischen Mechanismen, die der Speicherung des Sinapins in den Samen zugrundeliegen, sind bislang noch wenig untersucht. Es gilt jedoch als sicher, dass die vegetativen Pflanzenteile nicht als Quelle für die Sinapinsäure der Samen in Frage kommen, da sie dort nicht nachweisbar sind. So haben Bopp und Luedicke (Z. Naturforsch. 1980, 35c: 539-543) gezeigt, dass nach Fütterung von ¹⁴C-Phenylalanin an unreife Senf-Embryonen radioaktives ¹⁴C in Sinapin eingebaut wird. Daraus kann geschlossen werden, dass alle Enzyme der Hydroxyzimtsäure-Biosynthese von der Phenylalanin-Ammoniak-Lyase (PAL) bis zur Sinapinsynthase in den Samen-Embryonen aktiv sind.

Auch der Katabolismus des Sinapins während der Samenkeimung und Keimlingsentwicklung ist für Vertreter der Brassicaceen gut untersucht worden (A. Tzagoloff (1963), Plant. Physiol. 38: 202-206; M. Bopp und W. Luedicke (1975), Z. Naturforsch. 30c: 663-667; D. Strack (1977), Z. Pflanzenphysiol. 84: 139-145). Sinapin wird in keimenden Samen durch eine Sinapinesterase (G. Nurmann und D. Strack (1979), Z. Naturforsch. 34c: 715-720; D. Strack et al. (1980), Z. Naturforsch. 35c : 963-966) hydrolysiert und dient als Cholinquelle für die Membranlipide der jungen Keimpflanzen (D. Strack (1981), Z. Naturforsch. 36c: 215-221). Die Sinapinsäure wird über Sinapoylglucose als Intermediat in Sinapoylmalat überführt (D. Strack (1977)).

Ob Sinapin eine essentielle Bedeutung als Reservestoffbesitzt, ist noch nicht völlig geklärt. Chapple et al. (1992, The Plant Cell 4 : 1413-1424) untersuchten Arabidopsis-Mutanten mit stark reduziertem Sinapingehalt (5% des Wildtyps). Diese Mutanten akkumulierten vorwiegend freies Cholin anstelle des Cholinesters. Die Keimungsrate und das Wachstum der Mutanten war mit dem Wildtyp vergleichbar. Aus diesen Ergebnissen kann geschlossen werden, dass Sinapin kein essentieller Bestandteil des Samen für die Samenkeimung bzw. Keimlingsentwicklung ist.

Eine Aufgabe der vorliegenden Erfindung ist somit die Bereitstellung von transgenen Pflanzen, die einen im Vergleich mit ihrer Wildtypform verringerten Gehalt an Sinapin aufweisen und damit zur Proteingewinnung für Ernährungszwecke besser geeignet sind.

Die Entwicklung von Rapssorten mit niedrigem Sinapingehalt durch klassische Züchtungsprogramme war bislang wenig erfolgreich. Obwohl die verfügbaren Sorten teilweise deutliche Unterschiede in dem Gehalt an phenolischen Sekundärstoffe und in der Sinapinkonzentration zeigten, ist eine Selektion auf niedrigen Sinapingehalt sehr zeitaufwendig und wenig reproduzierbar.

Deswegen ist es eine weitere Aufgabe der vorliegenden Erfindung, den Sinapingehalt in Pflanzen durch genetische Unterdrückung von Enzymaktivitäten des Sinapin-Biosyntheseweges zu vermindern.

Ausgehend von der Sinapinsäure, die in den unreifen Samen aus dem Hydroxyzimtsäure-Biosyntheseweg angeliefert wird, werden für die Sinapinbildung zwei Enzyme benötigt, nämlich die UDP-Glucose:Sinapinsäure-Glucosyltransferase (SGT) (Nurmann und Strack (1981), supra) und die Sinapoylglucose:Cholin-Sinapoyltransferase (Sinapinsynthase; SCT) (Strack et al., (1983), supra):
SGT: Sinapinsäure + UDP-Glucose → Sinapoylglucose + UDP.
SCT: Sinapoylglucose + Cholin → Sinapin + Glucose.

Somit ist es eine weitere Aufgabe der vorliegenden Erfindung, die endogene SGT-Aktivität in transgenen Pflanzen, insbesondere in transgenen Ölsaaten und besonders bevorzugt in transgenen Rapspflanzen zu reduzieren oder vollständig zu blockieren.

Die für eine SGT oder SCT kodierenden Gensequenzen sind nicht bekannt. Die SCT konnte in Samen von 44 Vertretern der Brassicaceen nachgewiesen und eine Korrelation zwischen SCT-Aktivität und Sinapingehalt gezeigt werden (J. Regenbrecht und D. Strack (1985), Phytochemistry 24: 407-410). Die Reinigung der SGT aus *Brassica napus*-Keimlingen wurde kürzlich von Wang und Ellis (Phytochemistry (1998) 49: 307-318) beschrieben. Ferner wurde eine Teilreinigung dieses Enzyms beschrieben (H.-P. Mock und D. Strack (1993), Phytochemistry 92: 575-579). Jedoch sind weder von der SCT noch von der SGT bislang brauchbare Sequenzdaten ermittelt worden.

Der vorliegenden Erfindung liegt somit ferner die Aufgabe zugrunde, Verfahren zur Beeinflussung des Sinapingehalts in Pflanzen sowie rekombinante DNA-Moleküle zur Verfügung zu stellen, die eine DNA-Sequenz enthalten, die zur Manipulation des Sinapingehalts eingesetzt werden kann. Die vorstehend genannten und weitere Aufgaben der Erfindung werden durch die Bereitstellung der in den Patentansprüchen gekennzeichneten Ansführungsformen gelöst.

Im Rahmen der vorliegenden Erfindung wird jetzt erstmals eine DNA-Sequenz offenbart, die ein Protein mit der enzymatischen Aktivität einer SGT kodiert. Vorzugsweise stammt die Sequenz aus *Brassica,* besonders bevorzugt aus *Brassica napus.*

Durch Bereitstellung der erfindungsgemäßen DNA-Sequenzen kann die SGT beispielsweise durch Übertragung von Antisense- oder Sense- bzw. Cosuppressionskonstrukten der erfindungsgemäßen DNA-Sequenzen wirksam gehemmt werden und somit die Produktion von Sinapin im Samen unterbrochen werden. Des weiteren eignet sich für die Suppression der SGT-Aktivität in transgenen Pflanzen besonders die "double strength RNA interference"-Technik, auch als "post-transcriptional gene silencing" (PTGS). Im Unterschied zur klassischen "antisense"-Technik basiert die double strength RNA interference"-Methode auf der Bildung eines stabilen RNA-Doppelstranges des zu supprimierenden Gens in der Pflanzenzelle und führt zu einer im Vergleich zur normalen "antisense"-Technik mindestens genauso effizienten Suppression des spezifischen Targetgens. Für optimale Doppelstrangbildung wird empfohlen, zwischen sense- und antisense-Fragment ein Intron einzubauen.

Die Beobachtung, dass der Sinapingehalt durch die Inhibierung der SGT wirksam vermindert werden kann, lässt sich in idealer Weise für die Erzeugung sinapinarmer bzw. -freier Pflanzentransformanten einsetzen, die zur Proteingewinnung für Ernährungszwecke geeignet sind. Grundvoraussetzung für die Erzeugung solcher Nutzpflanzen ist die Verfügbarkeit geeigneter Transformationssysteme. Hier wurde während der letzten zwei Jahrzehnte ein breites Spektrum an Transformationsmethoden entwickelt und etabliert. Diese Techniken umfassen die Transformation pflanzlicher Zellen mit T-DNA unter Verwendung von *Agrobacterium tumefaciens* oder *Agrobacterium rhizogenes* als Transformationsmittel, die Fusion von Protoplasten, den direkten Gentransfer isolierter DNA in Protoplasten, die Injektion und Elektroporation von DNA in Pflanzenzellen, die Einbringung von DNA mittels biolistischer Methoden sowie weitere Möglichkeiten.

Neben der Bereitstellung der erfindungsgemäßen SGT-DNA-Sequenzen kann der Fachmann weitere SGT kodierende DNA-Sequenzen aus anderen Organismen mittels herkömmlicher molekularbiologischer Techniken selbst auffinden und im Rahmen der vorliegenden Erfindung einsetzen. So kann der Fachmann beispielsweise geeignete Hybridisierungssonden von den erfindungsgemäßen SGT-Sequenzen ableiten und für das Screening von cDNA- und/oder genomischen Banken des jeweils gewünschten Organismus, aus dem ein neues SGT-Gen isoliert werden soll, einsetzen. Hierbei kann der Fachmann auf geläufige Hybridisierungs-, Klonierungs- und Sequenzierungsmethoden zurückgreifen, die in jedem bio- oder gentechnologischem Labor wohl bekannt und etabliert sind (s. beispielsweise Sambrook et al. (1989) Molecular Cloning: A Laboratory Manual, 2. Auflage, Cold Spring Harbour Laboratory Press, Cold Spring Harbour, New York). Der Fachmann kann natürlich auch anhand der erfindungsgemäßen Sequenzen geeignete Oligonukleotidprimer für PCR-Amplifikationen von SGT-Sequenzen synthetisieren und einsetzen.

Bei einer wesentlichen Ausführungsform der vorliegenden Erfindung liegt die erfindungsgemäße, eine SGT kodierende DNA-Sequenz in der Antisense-Orientierung vor. Mit Hilfe der Antisense-Technik kann das endogene SGT-Gen bzw. -Gene gezielt inaktiviert werden, indem die erfindungsgemäße DNA-Sequenz "verkehrt" herum, also in Antisense-Orientierung in einen Vektor ligiert wird. Die RNA, die bei der Transkription eines derartigen Antisense-Gens entsteht, ist komplementär zur RNA des normalen Gens und kann die Synthese des SGT-Proteinprodukts verhindern, indem es zur Hybridisierung zwischen der nativen und der Antisense-RNA kommt.

Alternativ kann die endogene SGT-Expression durch ein Cosuppressionskonstrukt des erfindungsgemäßen Nukleinsäuremoleküls gehemmt werden. Bei der Cosuppression wird ein voraktives Gen durch zusätzlich eingeführte, identische Kopien dieses Gens inaktiviert. Diese Inaktivierung ist vermutlich auf einen RNA-abhängigen Mechanismus zurückzuführen, an dem die RNA-dirigierte RNA-Polymerase beteiligt ist

Wie oben erwähnt, besteht ein weiterer bevorzugter Suppressionsansatz in der Anwendung der PTGS-Methode, die auf der Bildung eines stabilen RNA-Doppelstranges des zu supprimierenden Gens, also des endogenen SGT-Gens in der Pflanzenzelle basiert.

Für den Fachmann ist klar, dass für das Erreichen eines Suppressionseffekts, der in einer Inhibierung der endogenen SGT-Aktivität resultiert, nicht zwingend die vollständige für SGT kodierende DNA-Sequenz eingesetzt werden muss. Die Erfindung betrifft somit auch Fragmente der erfindungsgemäßen DNA-Sequenzen, deren Verwendung innerhalb eines Antisense-, Sense- oder PTGS-Konstrukts in einer verringerten Aktivität des Ziel-SGT-Enzyms resultiert. Man könnte solche Fragmente im Zusammenhang mit dieser Erfindung auch als "Antisense-, Suppressions- oder PTGS-aktive" DNA-Fragmente bezeichnen, d.h. ihre Übertragung in Form eines geeigneten Konstrukts bewirkt eine Reduktion der endogenen SGT-Enzymaktivität.

Dem Fachmann ist es mittels üblicher Techniken auf einfache Weise durch Übertragung verschiedener Fragmente möglich, festzustellen, welche Länge bzw. welche Teilbereiche der vollständigen SGT-kodierenden Sequenz das Fragment aufweisen muss, um für die Inhibierung der endogenen Enzymaktivität geeignet zu sein.

Weiter bedarf es zur Ausführung der vorliegenden Erfindung lediglich geeigneter regulatorischer Sequenzen, die die Transkription einer operativ verknüpften SGT-DNA-Sequenz in den Samen der transformierten Pflanzen steuern. Auch hier kann der Fachmann geeignete Sequenzen problemlos dem Stand der Technik entnehmen oder selbst samenspezifische Promotorsequenzen isolieren. Beispiele für regulatorische Sequenzen, die für eine samenspezifische Transkription der erfindungsgemäßen Sequenzen bzw. Fragmenten davon besonders geeignet sind, sind der USP-Promotor aus *Vicia faba* (Bäumlein H. et al. (1991) Mol. Gen. Genet. 225: 459-467; Bäumlein H. et al. (1991) Mol. Gen. Genet. 225: 121-128), der LeB4-Promotor aus *Vicia faba* (Nagy I. (1990) Untersuchungen zur Expression und Regulation samenspezifischer Gene von *Vicia faba"* Dissertation, Universität Halle) und der DC3-Promotor aus *Daucus carota* (Steffens W.S. et al. (1990) Dev. Genet. 11 (1): 65-76). Eine weitere bevorzugte Ausführungsform stellt der napin-Promotor dar.

Besonders bevorzugt handelt es sich bei den regulatorischen Sequenzen um solche eines USP- oder LeB4-Promotors.

Diese samenspezifischen Promotoren können sowohl für Antisense-, Sense- als auch PTGS-Konstrukte eingesetzt werden, um Veränderungen des Sinapin-Biosynthesewegs mit dem Ziel einer Verminderung des Sinapingehalts in den Samen zu erreichen.

Obwohl eine samenspezifische Expression bevorzugt wird, kommt für die Expression der erfindungsgemäßen DNA-Sequenz in pflanzlichen Zellen jede Art von regulatorischer Sequenz in Frage, die die Transkription in pflanzlichen Zellen gewährleisten. Hier ist jeder in pflanzlichen Zellen aktive Promotor eingeschlossen. Dabei kann der Promotor so gewählt sein, dass die Expression konstitutiv erfolgt oder nur in Samengewebe zu einem bestimmten Zeitpunkt der Pflanzenentwicklung und/oder zu einem durch äußere Einflüsse, biotische oder abiotische Stimuli bestimmten Zeitpunkt (induzierte Genexpression). In bezug auf die zu transformierende Pflanze kann der Promotor homolog oder heterolog sein. Bei Verwendung eines konstitutiven Promotors kann eine zell- bzw. gewebespezifische Expression auch dadurch erreicht werden, dass die Genexpression in den Zellen bzw. Geweben, in denen sie nicht erwünscht ist, gehemmt wird, beispielsweise durch Ausprägung von Antikörpern, die das Genprodukt binden und somit seine Enzymaktivität unterbinden, oder durch geeignete Inhibitoren.

Samenspezifische Gene bzw. Promotoren kann der Fachmann dem Stand der Technik, insbesondere den einschlägigen wissenschaftlichen Journalen und Gendatenbanken entnehmen. Darüber hinaus ist der Durchschnittsfachmann in der Lage, mittels Routinemethoden weitere geeignete Promotoren zu isolieren. So kann der Fachmann mit Hilfe gängiger molekularbiologischer Methoden, z.B. Hybridisierungsexperimenten oder DNA-Protein-Bindungsstudien, samenspezifische regulatorische Nukleinsäureelemente identifizieren. Dabei wird z.B. in einem ersten Schritt aus Samengewebe des gewünschten Organismus, aus dem die regulatorischen Sequenzen isoliert werden sollen, die gesamte PolyA⁺-RNA isoliert und eine cDNA-Bank angelegt. In einem zweiten Schritt werden mit Hilfe von cDNA-Klonen, die auf PolyA⁺-RNA-Molekülen aus einem Nicht-Samengewebe basieren, aus der ersten Bank mittels Hybridisierung diejenigen Klone identifiziert, deren korrespondierende PolyA⁺-RNA-Moleküle lediglich in Samengewebe akkumulieren. Anschließend werden mit Hilfe dieser so identifizierten cDNAs Promotoren isoliert, die über samenspezifische regulatorische Elemente verfügen. Dem Fachmann stehen darüber hinaus weitere, auf PCR basierende Methoden für die Isolierung geeigneter samenspezifischer Promotoren zur Verfügung.

Ferner sind optional Transkriptions- bzw. Terminationssequenzen vorhanden, die der korrekten Beendigung der Transkription dienen, sowie der Addition eines PolyA-Schwanzes an das Transkript dienen können, dem eine Funktion bei der Stabilisierung der Transkripte beigemessen wird. Derartige Elemente sind in der Literatur beschrieben und beliebig austauschbar.

Schließlich erfolgt die Herstellung chimärer Genkonstrukte, in denen SGT-kodierende DNA-Sequenzen unter Kontrolle von regulatorischen Sequenzen stehen, die eine samenspezifische Transkription gewährleisten, mittels konventioneller Klonierungsmethoden (siehe beispielsweise Sambrook et al. (1989), supra). Die vorliegende Erfindung betrifft somit ein rekombinantes Nukleinsäuremolekül, umfassend:
a) regulatorische Sequenzen eines in Pflanzen, insbesondere in Pflanzensamen aktiven Promotors;
b) operativ daran gebunden eine DNA-Sequenz, die ein Protein mit der enzymatischen Aktivität einer SGT kodiert; oder ein Fragment der DNA-Sequenz, das für eine Suppression der endogenen SGT-Enzymaktivität ausreicht, und
c) ggf. operativ daran gebunden regulatorische Sequenzen, die als Transkriptions-, Terminations- und/oder Polyadenylierungssignale in Pflanzenzellen dienen können.

Wie oben erläutert, kann die operative Verknüpfung der SGT-DNA-Sequenz (b) mit den regulatorischen Sequenzen (a) in Sense- oder Antisense-Orientierung der SGT-DNA-Sequenz erfolgen.

Die DNA-Sequenz, die ein Protein mit der enzymatischen Aktivität einer SGT kodiert, kann aus natürlichen Quellen isoliert oder nach bekannten Verfahren synthetisiert werden. Vorzugsweise stammt die erfindungsgemäße DNA-Sequenz aus *Brassica,* insbesondere aus *Brassica napus.*

Mittels gängiger molekularbiologischer Techniken (siehe beispielsweise Sambrook et al. (1989), supra), ist es möglich, gewünschte Konstrukte für die Transformation von Pflanzen vorzubereiten bzw. herzustellen. Die für die gentechnische Manipulation in prokaryontischen Zellen üblicherweise eingesetzten Klonierungs-, Mutagenisierungs-, Sequenzanalyse-, Restriktionsanalyse- und weitere biochemischmolekularbiologische Methoden sind dem Durchschnittsfachmann wohl bekannt. So können nicht nur geeignete chimäre Genkonstrukte mit der gewünschten Fusion von Promotor und SGT-DNA-Sequenz hergestellt werden, vielmehr kann der Fachmann mittels Routinetechniken, falls erwünscht, verschiedenartige Mutationen oder Deletionen in die SGT-kodierende DNA-Sequenz einführen.

In einer bevorzugten Ausführungsform ist die DNA-Sequenz, die ein Protein mit der enzymatischen Aktivität einer SGT kodiert, ausgewählt aus der Gruppe, bestehend aus:
a) DNA-Sequenzen, die eine Nukleotidsequenz umfassen, die die in SEQ ID No. 3 angegebene Aminosäuresequenz oder Fragmente davon kodieren;
b) DNA-Sequenzen, die die in SEQ ID No. 1 angegebene Nukleotidsequenz oder Teile davon umfassen;
c) DNA-Sequenzen, die eine Nukleotidsequenz, die mit einem komplementären Strang der Nukleotidsequenz von a) oder b) hybridisieren, oder Teile dieser Nukleotidsequenz umfassen;
d) DNA-Sequenzen, die eine Nukleotidsequenz, die zu einer Nukleotidsequenz von c) degeneriert ist, oder Teile dieser Nukleotidsequenz umfassen;
e) DNA-Sequenzen, die ein Derivat, Analog oder Fragment einer Nukleotidsequenz von a), b), c) oder d) darstellen.

Der Begriff "Hybridisierung" bedeutet im Rahmen der vorliegenden Erfindung eine Hybridisierung unter konventionellen Hybridisierungsbedingungen, vorzugsweise unter stringenten Bedingungen, wie sie beispielsweise in Sambrook et al. (1989, supra) beschrieben sind. Geeignete stringente Bedingungen schließen Salzlösungen von etwa 0,9 molar bei Temperaturen von 35°C bis 65°C ein. Bevorzugt schließen stringente Hybridisierungsbedingungen folgende Bedingungen ein:

| | |
|---|---|
| Hybridisierungspuffer: | 7 % SDS 250 mM NaCl 250 mM K-Phosphat-Puffer pH 7,0 1 mM EDTA |
| Hybridisierungstemperatur: | 58°C bis 60 °C |
| Hybridisienmgszeit: | über Nacht |
| Waschpuffer: | I 2 x SSC 0,1 % SDS |
| | II 0,2 x SSC 0,1 % SDS |
| Waschtemperatur und-zeit: | jeweils 2 x 30 min. bei 55°C bis 60°C |

DNA-Sequenzen, die mit den DNA-Sequenzen, die ein Protein mit der enzymatischen Aktivität einer SGT kodieren, hybridisieren, können z.B. aus genomischen oder cDNA-Bibliotheken isoliert werden. Die Identifizierung und Isolierung derartiger DNA-Sequenzen kann z.B. unter Verwendung von DNA-Sequenzen erfolgen, die exakt oder im wesentlichen eine der oben erwähnten SGT-kodierenden Nukleotidsequenzen oder Teile dieser Sequenzen aufweisen, bzw. der reversen Komplemente dieser DNA-Sequenzen, z.B. mittels Hybridisierung nach Standardverfahren (s. z.B. Sambrook et al. (1989), supra). Bei den als Hybridisierungssonde verwendeten Fragmenten kann es sich auch um synthetische Fragmente handeln, die mit Hilfe üblicher Synthesetechniken hergestellt wurden und deren Sequenz im wesentlichen mit einer der oben erwähnten DNA-Sequenzen für SGT oder einem Teil einer dieser Sequenzen übereinstimmt. Die DNA-Sequenzen, die ein Protein mit der enzymatischen Aktivität einer SGT kodieren, umfassen auch DNA-Sequenzen, deren Nukleotidsequenz zu einer der vorstehend beschriebenen DNA-Sequenzen degeneriert ist.

Die erfindungsgemäßen DNA-Sequenzen umfassen auch Fragmente, Derivate und allelische Varianten der oben beschriebenen DNA-Sequenzen, die ein Protein mit der enzymatischen Aktivität einer SGT kodieren. Der Ausdruck "Derivat" bedeutet in diesem Zusammenhang, dass die Sequenzen sich von den oben beschriebenen DNA-Sequenzen an einer oder mehreren Positionen unterscheiden, aber einen hohen Grad an Homologie zu diesen Sequenzen aufweisen. Homologie bedeutet dabei, eine Sequenzidentität von mindestens 40%, insbesondere eine Identität von mindestens 60%, 70%, vorzugsweise von mindestens 80%, 82%, 84%, 86%, 88%, und besonders bevorzugt von mindestens 90%, 92%, 94%, 96%, 98%. Die Abweichungen zu den oben beschriebenen DNA-Sequenzen können dabei beispielsweise durch Deletion, Substitution, Insertion oder Rekombination entstanden sein.

Bei den DNA-Sequenzen, die homolog zu den oben beschriebenen Sequenzen sind und Derivate dieser Sequenzen darstellen, handelt es sich in der Regel um Variationen dieser Sequenzen, die Modifikationen darstellen, die dieselbe biologische Funktion ausüben. Es kann sich dabei sowohl um natürlicherweise auftretende Variationen handeln, beispielsweise um Sequenzen aus anderen Organismen, oder um Mutationen, wobei diese Mutationen auf natürliche Weise aufgetreten sein können oder durch gezielte Mutagenese eingeführt wurden. Ferner kann es sich bei den Variationen um synthetisch hergestellte Sequenzen handeln. Bei den allelischen Varianten kann es sich sowohl um natürlich auftretende Varianten handeln als auch um synthetisch hergestellte oder durch rekombinante DNA-Techniken erzeugte Varianten.

In einer besonders bevorzugten Ausführungsform stammt die beschriebene, für eine SGT kodierende DNA-Sequenz aus *Brassica napus* (wie in SEQ ID No. 1 angegeben).

Die von den erfindungsgemäßen DNA-Sequenzen kodierten Enzyme weisen eine Aminosäuresequenz auf, die mit der in SEQ ID No. 3 angegebenen Sequenz in mindestens 30%, 40% der Aminosäuren, insbesondere in mindestens 50%, 60%, 70%, vorzugsweise in mindestens 80%, 82%, 84%, 86%, 88%, und besonders bevorzugt in mindestens 90%, 92%, 94%, 96%, 98% der Aminosäuren übereinstimmt.

Die Erfindung betrifft weiterhin Vektoren und Mikroorganismen, die erfindungsgemäße Nukleinsäuremoleküle enthalten und deren Verwendung die Herstellung von Pflanzenzellen und Pflanzen ermöglicht, deren Sinapingehalt gegenüber Wildtyp-Pflanzenzellen bzw. -Pflanzen verringert ist. Dabei handelt es sich bei den Vektoren insbesondere um Plasmide, Cosmide, Viren, Bakteriophagen und andere in der Gentechnik gängige Vektoren. Bei den Mikroorganismen handelt es sich in erster Linie um Bakterien, Viren, Pilze, Hefen und Algen.

Des weiteren wird bei der vorliegenden Erfindung ein rekombinantes Protein mit der enzymatischen Aktivität einer SGT aus *Brassica,* insbesondere ein rekombinantes Protein mit der in SEQ ID No. 3 angegebenen Aminosäuresequenz bereitgestellt.

Weiter betrifft die Erfindung ein Verfahren zur Erzeugung von Pflanzen bzw. Pflanzenzellen mit einem gegenüber Wildtyp-Pflanzen bzw. -pflanzenzellen verringerten Sinapingehalt, umfassend die folgenden Schritte:
a) Herstellung eines rekombinanten Nukleinsäuremoleküls, das folgende Sequenzen umfasst:
   - regulatorische Sequenzen eines in Pflanzen aktiven Promotors;
   - operativ daran gebunden eine DNA-Sequenz, die ein Protein mit der enzymatischen Aktivität einer SGT kodiert; und
   - optional operativ daran gebunden regulatorische Sequenzen, die als Transkriptions-Terminations- und/oder Polyadenylierungssignale in Pflanzenzellen dienen können;
b) Übertragung des Nukleinsäuremoleküls aus a) auf pflanzliche Zellen; und
c) gegebenenfalls die Regeneration intakter Pflanzen aus den transformierten Pflanzenzellen.

Die DNA-Sequenz, die ein Protein mit der enzymatischen Aktivität einer SGT kodiert, liegt bei einer besonders bevorzugten Ausführungsform der vorliegenden Erfindung als Antisense-, Sense- bzw. Cosuppressionskonstrukt oder als PTGS-Konstrukt vor, um die Expression der SGT in der transformierten Pflanze bzw. Pflanzenzelle zu hemmen bzw. vollständig zu unterdrücken und somit eine Reduzierung des Sinapingehalts in den transformierten Pflanzen herbeizuführen.

Ein weiterer Aspekt der vorliegenden Erfindung ist, dass durch die Blockierung der Sinapin-Biosynthese die Verfügbarkeit der Cumarsäure, eines Intermediats der Sinapin-Biosynthese, gesteigert wird. Die Cumarsäure wird zur Biosynthese von Resveratrol benötigt, das ernährungsphysiologisch von Bedeutung ist. Ferner führt die Blockierung der Sinapin-Biosynthese zur Akkumulation von freiem Cholin. Dadurch wird als weiterer Vorteil der vorliegenden Erfindung die Verfügbarkeit des Cholins für die Lecithin-Biosynthese erhöht, was bei transformierten Ölsaaten zu einer Erhöhung des Fettsäuregehalts, z.B. von Palmitinsäure, Stearinsäure, Palmitoleinsäure, Oleinsäure, Linolsäure, Linolensäure führt.

Die Erfindung betrifft ferner Pflanzenzellen, die die erfindungsgemäßen Nukleinsäuremoleküle, die ein Protein mit der enzymatischen Aktivität einer SGT kodieren, enthalten. Die Erfindung betrifft ebenfalls Ernteprodukte und Vermehrungsmaterial transgener Pflanzen sowie die transgenen Pflanzen selbst, die ein erfindungsgemäßes Nukleinsäuremolekül enthalten. Die transgenen Pflanzen der vorliegenden Erfindung weisen aufgrund der Einführung einer SGT-kodierenden DNA-Sequenz vorzugsweise in Antisense-Orientierung oder als Cosuppressionskonstrukt in den Samen einen geringeren Gehalt an Sinapin auf.

Bei einem weiteren Aspekt der vorliegenden Erfindung können aus den mit einer erfindungsgemäßen SGT-DNA-Sequenz transformierten Pflanzen pflanzliches Protein gewonnen werden, das einen gegenüber aus Wildtyp-Pflanzen gewonnenem Protein verringerten Sinapingehalt aufweist und somit vorteilhaft für Ernährungszwecke eingesetzt werden kann.

Bei einer bevorzugten Ausführungsform der vorliegenden Erfindung wird das pflanzliche Protein mit einem verringerten Sinapingehalt aus mit einer erfindungsgemäßen SGT-DNA-Sequenz in Antisense-Orientierung oder als Cosuppressions-Konstrukt transformiertem Raps gewonnen.

Zur Vorbereitung der Einführung fremder Gene in höhere Pflanzen bzw. deren Zellen stehen eine große Anzahl von Klonierungsvektoren zur Verfügung, die ein Replikationssignal für *E*. *coli* und ein Markergen zur Selektion transformierter Bakterienzellen enthalten. Beispiele für derartige Vektoren sind pBR322, pUC-Serien, M13mp-Serien, pACYC184 usw. Die gewünschte Sequenz kann an einer passenden Restriktionsschnittstelle in den Vektor eingeführt werden. Das erhaltene Plasmid wird dann für die Transformation von *E. coli*-Zellen verwendet. Transformierte *E. coli-*Zellen werden in einem geeigneten Medium gezüchtet und anschließend geerntet und lysiert, und das Plasmid wird wiedergewonnen. Als Analysenmethode zur Charakterisierung der gewonnenen Plasmid-DNA werden im allgemeinen Restriktionsanalysen, Gelelektrophoresen und weitere biochemischmolekularbiologische Methoden eingesetzt. Nach jeder Manipulation kann die Plasmid-DNA gespalten und gewonnene DNA-Fragmente mit anderen DNA-Sequenzen verknüpft werden.

Für die Einführung von DNA in eine pflanzliche Wirtszelle stehen eine Vielzahl von Techniken zur Verfügung, wobei der Fachmann die jeweils geeignete Methode ohne Schwierigkeiten ermitteln kann. Diese Techniken umfassen, wie bereits oben erwähnt, die Transformation pflanzlicher Zellen mit T-DNA unter Verwendung von *Agrobacterium tumefaciens* oder *Agrobacterium rhizogenes* als Transformationsmedium, die Fusion von Protoplasten, die Injektion, die Elektroporation, den direkten Gentransfer isolierter DNA in Protoplasten, die Einbringung von DNA mittels der biolistischen Methode sowie weitere Möglichkeiten die bereits seit mehreren Jahren gut etabliert sind und zum üblichen Repertoire des Fachmanns in der pflanzlichen Molekularbiologie bzw. Pflanzenbiotechnologie gehören.

Bei der Injektion und Elektroporation von DNA in Pflanzenzellen werden per se keine speziellen Anforderungen an die verwendeten Plasmide gestellt. Ähnliches gilt für den direkten Gentransfer. Es können einfache Plasmide, wie z.B. pUC-Derivate, verwendet werden. Sollen aber aus derartig transformierten Zellen ganze Pflanzen regeneriert werden, ist die Anwesenheit eines selektierbaren Markergens empfehlenswert. Dem Fachmann sind die gängigen Selektionsmarker bekannt, und es stellt für ihn kein Problem dar, einen geeigneten Marker auszuwählen.

Je nach Einführungsmethode gewünschter Gene in die Pflanzenzelle können weitere DNA-Sequenzen erforderlich sein. Werden z.B. für die Transformation der Pflanzenzelle das Ti- oder Ri-Plasmid verwendet, so muss mindestens die rechte Begrenzung, häufig jedoch die rechte und linke Begrenzung der im Ti- bzw. Ri-Plasmid enthaltenen T-DNA als Flankenbereich mit den einzuführenden Genen verbunden werden. Werden für die Transformation Agrobakterien verwendet, muss die einzuführende DNA in spezielle Plasmide kloniert werden, und zwar entweder in einen intermediären oder in einen binären Vektor. Die intermediären Vektoren können aufgrund von Sequenzen, die homolog zu Sequenzen in der T-DNA sind, durch homologe Rekombination in das Ti- oder Ri-Plasmid der Agrobakterien integriert werden. Dieses enthält außerdem die für den Transfer der T-DNA notwendige vir-Region. Intermediäre Vektoren können nicht in Agrobakterien replizieren. Mittels eines Helferplasmids kann der intermediäre Vektor auf *Agrobacterium tumefaciens* übertragen werden (Konjugation). Binäre Vektoren können sowohl in *E. coli* als auch in Agrobakterien replizieren. Sie enthalten ein Selektionsmarkergen und einen Linker oder Polylinker, welche von der rechten und linken T-DNA-Grenzregion eingerahmt werden. Sie können direkt in die Agrobakterien transformiert werden. Das als Wirtszelle dienende Agrobakterium soll ein Plasmid, das eine vir-Region trägt, enthalten. Die vir-Region ist für den Transfer der T-DNA in die Pflanzenzelle notwendig. Zusätzliche T-DNA kann vorhanden sein. Das derartig transformierte Agrobakterium wird zur Transformation von Pflanzenzellen verwendet. Die Verwendung von T-DNA für die Transformation von Pflanzenzellen ist intensiv untersucht und ausreichend in allseits bekannten Übersichtsartikeln und Handbüchern zur Pflanzentransformation beschrieben worden. Für den Transfer der DNA in die Pflanzenzelle können Pflanzen-Explantate zweckmäßigerweise mit *Agrobacterium tumefaciens* oder *Agrobacterium rhizogenes* kultiviert werden. Aus dem infizierten Pflanzenmaterial (z.B. Blattstücke, Stengelsegmente, Wurzeln, aber auch Protoplasten oder Suspensions-kultivierte Pflanzenzellen) können dann in einem geeigneten Medium, welches Antibiotika oder Biozide zur Selektion transformierter Zellen enthalten kann, wieder ganze Pflanzen regeneriert werden.

Ist die eingeführte DNA einmal im Genom der Pflanzenzelle integriert, so ist sie dort in der Regel stabil und bleibt auch in den Nachkommen der ursprünglich transformierten Zelle erhalten. Sie enthält normalerweise einen Selektionsmarker, der den transformierten Pflanzenzellen Resistenz gegenüber einem Biozid oder einem Antibiotikum wie Kanamycin, G 418, Bleomycin, Hygromycin, Methotrexat, Glyphosat, Streptomycin, Sulfonylharnstoff, Gentamycin oder Phosphinotricin u.a. vermittelt.

Der individuell gewählte Marker sollte daher die Selektion transformierter Zellen gegenüber Zellen, denen die eingeführte DNA fehlt, gestatten. Hierzu sind auch alternative Marker geeignet, wie nutritive Marker, Screeningmarker (wie GFP, green fluorescent protein). Selbstverständlich kann auch vollkommen auf Selektionsmarker verzichtet werden, was allerdings mit einem ziemlich hohen Screeningbedarf einhergeht. Falls markerfreie transgenen Pflanzen erwünscht sind, stehen dem Fachmann auch Strategien zur Verfügung, die eine nachträgliche Entfernung des Markergens erlauben, z.B. Cotransformation, Sequenz-spezifische Rekombinasen.

Die Regeneration der transgenen Pflanzen aus transgenen Pflanzenzellen erfolgt nach üblichen Regenerationsmethoden unter Verwendung bekannter Nährmedien. Die so erhaltenen Pflanzen können dann mittels üblicher Verfahren, einschließlich molekularbiologischer Methoden, wie PCR, Blot-Analysen, auf Anwesenheit der eingeführten Nukleinsäure, die ein Protein mit der enzymatischen Aktivität einer SGT kodiert, untersucht werden.

Bei der transgenen Pflanze bzw. den transgenen Pflanzenzellen kann es sich um jede beliebige monokotyle oder dikotyle Pflanze bzw. Pflanzenzelle handeln, die einen unerwünscht hohen Gehalt an Sinapin aufweist. Vorzugsweise handelt es sich um Nutzpflanzen bzw. Zellen von Nutzpflanzen. Besonders bevorzugt handelt es sich um Ölsaaten, hier ganz besonders bevorzugt um Raps oder Rübsen, Getreide, Zuckerrübe, Mais, Sonnenblume und Lein. Prinzipiell ist jede Nutzpflanze für die Umsetzung der Erfindung erstrebenswert, die für Ernährungszwecke geeignet ist.

Die Erfindung betrifft ebenfalls Vermehrungsmaterial und Ernteprodukte der erfindungsgemäßen Pflanzen, beispielsweise Früchte, Samen, Knollen, Wurzelstöcke, Sämlinge, Stecklinge usw.

Die spezifische Expression der SGT in den Samen der erfindungsgemäßen Pflanzen bzw. in den erfindungsgemäßen Pflanzenzellen kann mit Hilfe herkömmlicher molekularbiologischer und biochemischer Methoden nachgewiesen und verfolgt werden. Dem Fachmann sind diese Techniken bekannt und er ist problemlos in der Lage, eine geeignete Nachweismethode zu wählen, beispielsweise eine Northern-Blot-Analyse zum Nachweis SGT-spezifischer RNA bzw. zur Bestimmung der Höhe der Akkumulation von SGT-spezifischer RNA oder eine Southern-Blot-Analyse zur Identifizierung von für SGT-kodierende DNA-Sequenzen.

Ferner betrifft die Erfindung ein Verfahren zur Gewinnung von pflanzlichem Protein mit einem gegenüber Wildtyp-Pflanzenprotein verringerten Sinapingehalt, umfassend die folgenden Schritte:
a) die Übertragung eines rekombinanten Nukleinsäuremoleküls oder Vektors, der eine DNA-Sequenz enthält, die für ein Protein mit der enzymatischen Aktivität einer SGT kodiert, auf Pflanzenzellen;
b) die Regeneration von Pflanzen aus den transformierten Pflanzenzellen;
c) die Verarbeitung der Samen der Pflanzen aus b) zur Gewinnung von pflanzlichem Protein mit einem verringerten Gehalt an Sinapin.

Die vorliegende Erfindung wird in den nachfolgenden Beispielen, die nur der Veranschaulichung der Erfindung dienen und in keiner Weise als Einschränkung zu verstehen sind, erläutert.

### Beispiele

Aus Raps (*Brassica napus*) wurde eine cDNA isoliert, die für das Enzym UDP-Glucose:Sinapinsäure-Glucosyltransferase (SGT) kodiert. Wie bereits vorstehend erwähnt, katalysiert die SGT die Umsetzung von UDP-Glucose und Sinapinsäure zu 1-O-Sinapoylglucose (SinGlc), die wiederum als aktivierter Acyldonator für die Sinapin-Biosynthese fungiert.

### a) Selektionsmethode

Aus unreifen Samen und Kotyledonen von *Brassica napus*-Keimpflanzen der Sorte Express wurden cDNA-Banken angelegt. Als Vektor diente der modifizierte λ-Phage Uni-ZAP XR (Stratagene, La Jolla, CA, USA). Mit diesen cDNA-Banken als Template wurden mit Hilfe von PCR Glucosyltransferase-spezifische Sequenzen amplifiziert. Als PCR-Primer in "forward"-Orientierung dienten Oligonukleotide, deren Sequenzen von einer in Glucosyltransferasen des pflanzlichen Sekundärstoff wechsels hochkonservierten Region, der PSPG-Box abgeleitet sind (PSPG = "plant secondary product glucosyltransferase", Proteinsequenzmotiv der PSPG-Box: THCGWN; Hughes J. and Hughes M.A. (1994) DNA Sequence 5: 41-49). U.a. wurde als foreward-Primer folgendes Oligonukleotid eingesetzt: 5'-ACNCAYTGYGGNTGGAAC-3'. Als "Reverse-Primer" wurde der T7-Primer eingesetzt. Die PCR-Reaktion lief nach folgendem Protokoll ab (Eppendorf-Mastecycler) :

| | | |
|---|---|---|
| "hot start": | 94°C | 4 min. |
| 30 Zyklen: | 94°C | 1 min. (Denaturierung) |
| | 56°C | 1 min. (Annealing) |
| | 72°C | 2 min. (Synthese). |

Der Reaktionsansatz enthielt:

| | |
|---|---|
| dATP, dGTP, dCTP, dTTP: | je 200 µM |
| Primer (foreward PSPG, T7): | je 100 pMol |
| Raps-cDNA-Banken aus unreifen Samen bzw. Kotyledonen | je 3 x 10⁶ pfu* |
| Taq-DNA-Polymerase (Appligene): | 1 unit |
| Puffer für Taq-Polymerase | |
| Wasser | |
| (*pfu = plaques forming units) | |

Die über PCR amplifizierten Fragmente wurden mit α-[³²P]dATP markiert und als Sonden zur Hybridisierung gegen die *Brassica* napus-cDNA-Banken aus Samen und Kotyledonen eingesetzt. Positive Klone wurden isoliert, die rekombinanten pBluescript-Phagemide über die in vivo-"Excisions"-Technik (Stratagene) ausgeschnitten und in *E. coli* (XL1-Blue MRF'; Stratagene) transformiert.

Von allen auf diese Weise isolierten cDNA-Fragmenten aus *Brassica napus* wurde vom 5'-Ende her die DNA-Sequenz bestimmt. Eine der cDNA-Sequenzen konnte sowohl aus den Samen ("SP15") als auch aus den Cotyledonen ("CP5") isoliert werden, wobei sie in den Samen in höherer Abundanz auftritt. Diese Sequenz zeigte die höchste Identität zu einer "probable indole-3-acetate β-glucosyltransferase" (E71419) aus *Arabidopsis thaliana.* Ihr Leserahmen kodiert für ein Protein mit 497 Aminosäuren (siehe SEQ ID No. 3). Dieses Protein zeigte eine hohe SGT-Aktivität. Die kodierende Sequenz befindet sich in der Gendatenbank unter der Accession-No. AF287143, die Proteinsequenz unter der Accession-No. AAF98390; siehe auch Milkowski et al. (2000) Planta 211: 883-886.

### b) Funktioneller Test

Da in den rekombinanten pBluescript-Phagemiden die cDNA's "downstream" vom *E. coli-lac-*Promotor inseriert sind, wurden klonierte Gene im *E. coli*-Wirt exprimiert. Zum Test auf SGT-Aktivität wurden die *E. coli*-Zellen des Stammes XL1-Blue MRF, die Phagemide mit den Inserts "CPS" und "SP15" tragen, mit UDP-Glucose und Sinapinsäure inkubiert (60 min bei 32°C). Die Zellen wurden anschließend lysiert, die Extrakte mit Trifluoressigsäure gefällt und die Überstände mit Hilfe der HPLC analysiert. Das Elutionsprofil ergab in beiden Fällen das erwartete Reaktionsprodukt, das massenspektroskopisch (LC-MS) als Sinapoylglucose identifiziert werden konnte.

Damit wurde eindeutig gezeigt, dass die SGT-cDNA aus *Brassica napus* kloniert wurde.

### c) Tagging und Aufreinigung der SGT

Mittels PCR wurde die vollständige cDNA-Sequenz der SGT von *B. napus* amplifiziert (ohne Stopcodon), wobei Restriktionsschnittstellen für *Nco*I (am 5'-Ende) und *Bam*HI (am 3'-Ende) eingefügt wurden. Als Template diente der pBluescript-Vektor, der die gesamte SGT-cDNA aus *B. napus* enthält. Das erhaltene PCR-Fragment wurde in den *Nco*I- *Bam*HI-gespaltenen Vektor pQE60 (Qiagen, Hilden) kloniert. Die Konstrukte wurden in den *E. coli*-Stamm M15/pREP4 (Qiagen, Hilden) transformiert.

Der NcoI-Forward-Primer ist weiter unten angeben, gleiches gilt für den BamHIreverse-Primer.

### d) Anzucht der E. coli-Kulturen und Induktion der SGT-Expression

Die Zellen wurden in LB-Medium mit Ampicillin (100 µg/ml) und Kanamycin (25 µg/ml) in Schüttelkultur (Batch-Kultur; 400 ml) bei 37°C angezogen. In der frühen logarithmischen Wachstumsphase (OD600=0,3) wurde zur Induktion der Expression der rekombinanten SGT Isopropyl-β-D-thiogalaktosid (IPTG) zugegeben (23,8 mg/ml) und die Zellen anschließend über Nacht bei 30°C weiterkultiviert.

Durch Zentrifugation (4000 rpm, 10 min, 4°C) wurden die Zellen geerntet und umgehend bei - 80°C eingefroren.

### e) Proteinextraktion und Reinigung

*E. coli*-Zellen aus 121 Kultur (ca. 10 g Trockenmasse) wurden in 50 mM Phosphat-puffer, pH 8, der zusätzlich 300 mM NaCl und 10 mM Imidazol enthält (Aufschlußpuffer), suspendiert und durch Ultraschallbehandlung aufgeschlossen. Die spezifische SGT-Aktivität des Rohextrakts betrug 1,2 nkat/mg Protein. Der Extrakt wurde auf eine Kobaltagarosesäule (5 cm x 2,6 cm i. d., Flussrate 3 ml/min) gegeben. Nach dem Spülen der Säule mit Aufschlußpuffer wurde noch mit drei Säulenvolumen Waschpuffer (wie Aufschlußpuffer, aber mit 20 mM Imidazol) unspezifisch gebundenes Protein eluiert. Die Elution des gebundenen Proteins erfolgte mit Phosphat-puffer, der 0,25 M Imidazol enthielt. Die aktiven Fraktionen wurden vereinigt und mit 80 % (NH₄)₂SO₄ ausgefällt (Ausbeute 3,7 mg). Die weitere Reinigung der SGT erfolgte durch Gelfiltration (Superdex 200; 26/60). Die aktiven Fraktionen wurden vereinigt und mit Centricon-Filtereinheiten konzentriert (Ausbeute 1,2 mg Protein). Ein letzter Reinigungsschritt erfolgte durch hydrophobe Interaktion mit Phenylsuperose HR 5/5. Die Proteinausbeute nach dieser Reinigung betrug 570 µg, die spezifische Aktivität 400 nkat/mg Protein. Damit konnte eine Anreicherung auf das 400fache erreicht werden.

Die Bestimmung der Molmasse erfolgte durch SDS-Gelelektrophorese sowie Gelfiltration auf einer Superose 12 Säule. Die Gelelektrophorese ergab für die rekombinante SGT eine Molmasse von 62 kDa; mit der Gelfiltration wurden 60 kDa ermittelt.

### f) Enzymassay

Für die kinetischen Untersuchungen wurde das Eluat der Kobaltagarosesäule nach Fällung mit Ammoniumsulfat und Entsalzung eingesetzt. Zur Stabilisierung wurde 1 mg/ml Rinderserumalbumin zugegeben.

Der Enzymassay enthielt 20 mM UDP-Glukose, 900 ng Enzym und Sinapinsäure verschiedener Konzentration (0,05; 0,1; 0,2; 0,4; 0,6; 1; 2; 4 mM). Als Puffer wurde 0,1 M MES, pH 6,0, bei Inkubationszeiten von 2,5; 5 und 10 Minuten verwendet. Für Sinapinsäure wurde ein Km-Wert von 0,13 mM ermittelt.

### g) Herstellung transgener Pflanzen

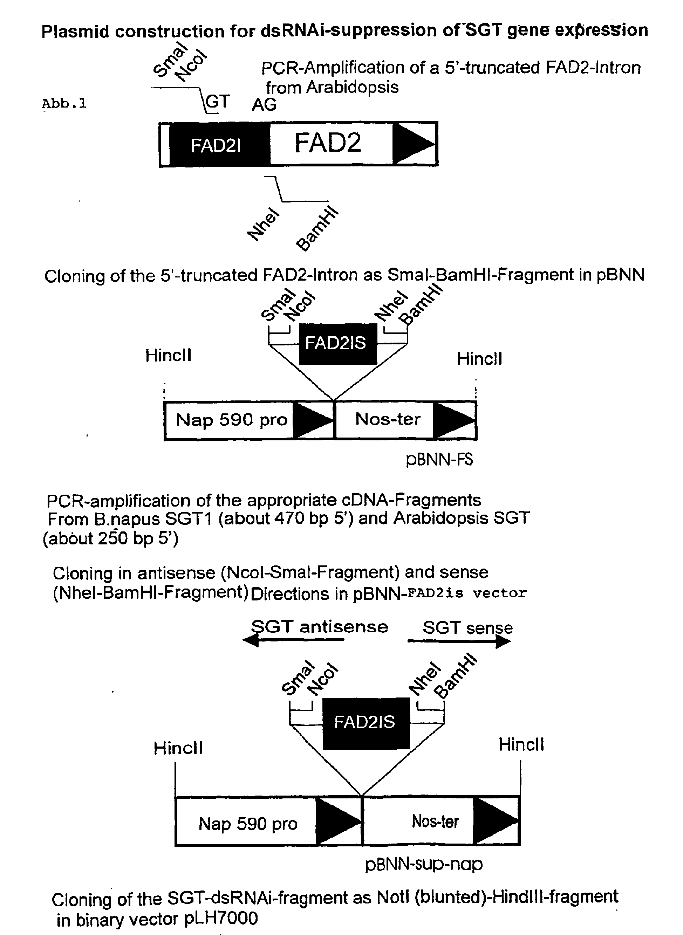

Die das SGT-Protein kodierende DNA-Sequenz wird in antisense-Orientierung mit einem samenspezifischen Promotor, vorzugsweise USP-, LeB4- oder napin-Promotor, fusioniert. Der Suppressionsansatz der double strand RNA interference (dsRNAI-Suppression) ist ebenfalls bevorzugt, dieser Ansatz ist in Abbildung 1 veranschaulicht.

Die Promotor-SGT-antisense-Fusion bzw. das dsRNAI-Suppressionskonstrukt wird auf einen für die Transformation von Raps geeigneten Vektor gebracht und das resultierende Konstrukt auf Raps übertragen. Von den transgenen Rapspflanzen werden unreife Samen geerntet. Im Rohextrakt wird über HPLC-Analyse der Gehalt an 1-*O*-Sinapoylglucose sowie an 1-*O*-Sinapoylcholin (Sinapin) bestimmt. Außerdem wird die SGT-Aktivität gemessen.

Konstruktion von Plasmiden für die Suppression der SGT-Expression in transgenen Pflanzen, insbesondere in *Brassica napus* und *Arabidopsis:*
Konstruktion eines Suppressionsvektors für *Brassica napus*
Klonierung eines verkürzten Introns aus Arabidopsis
Aus genomischer DNA von *Arabidopsis thaliana* wurde zunächst über PCR eine Sequenz aus dem Intron 1 des FAD2-Gens amplifiziert (Okuley et al. (1994) Plant Cell 6:147-158). Hierzu wurden folgende Primer eingesetzt:

Das amplifizierte Fragment enthält den 3'-Teil des FAD2-Introns (264 Nukleotide) und ist flankiert von den Restriktionsschnittstellen für SmaI und *Nco*I (am 5'-Ende) sowie *Nhe*I und *Bam*HI (am 3'-Ende).

Die amplifizierte Sequenz ist wie folgt:

(Die klein geschriebene Sequenz ist die Intronsequenz; groß geschriebene Nukleotide stammen aus den angefügten Primer-Sequenzen.)

Dieses Fragment wurde als *Sma*I-*Bam*HI-Fragment in den ebenfalls mit *Sma*I und BamHI verdauten Vektor pBNN kloniert. Der Vektor pBNN ist ein Derivat von pBIIKS, das den napin-Promotor 590 und den nos-Terminator trägt. Zwischen Promotor und Terminator befinden sich unikale Restriktionsschnittstellen für die Klonierung. Das resultierende Konstrukt pBNN-FS trägt das verkürzte FAD2-Intron zwischen nap-Promotor und nos-Terminator.

### Klonierung eines SGT-Fragments in antisense-Orientierung in pBNN-FS

Das Plasmid pB-SGT1, pBluescript SK mit der vollständigen cDNA der *Brassica napus* SGT1 als EcoRI-XhoI-Fragment, diente als Template, um über PCR ein spezifisches Fragment der SGTI-Sequenz zu amplifizieren. Es wurden folgende Primer eingesetzt:

Das amplifizierte Fragment enthält den 5'-Teil der kodierenden Region der SGT1-cDNA (470 Nukleotide, Beginn mit dem A des Startcodons) und ist flankiert von den Restriktionsschnittstellen für *Nco*I (5') und SmaI (3').

(Die groß geschriebene Sequenz ist die SGT1-Sequenz; klein geschriebene Nukleotide stammen aus den angefügten Primer-Sequenzen.)

Dieses Fragment wurde als *Nco*I-*Sma*I-Fragment in den *Nco*I*-* und *Sma*I-gespaltenen Vektor pBNN-FS kloniert. Das resultierende Konstrukt pBNN-SGT-FS enthält die SGT-Sequenz in antisense-Orientierung zwischen nap-Promotor (nap = Napin) und verkürztem FAD2-Intron.

### Klonierung des SGT-Fragments in sense-Orientierung in pBNN-SGT-FS

Dieselbe SGT1-Sequenz wie oben für die Klonierung in antisense-Orientierung in pBNN-FS beschrieben wurde über PCR mit folgenden Primern amplifiziert:

Das Fragment wurde als *Nhe*I*-Bam*HI*-*Fragment in den *Nhe*I-*Bam*HI-gespaltenen Vektor pBNN-SGT-FS kloniert.

Das resultierende Konstrukt pBNN-sup-nap enthält folgende Kassette (Suppressionskassette): nap 590-Promotor- SGT1 (470 bp antisense) verkürztes FAD2-Intron-SGT1(470bp sense)-nos-Terminator.

### Klonierung der Suppressionskassette aus pBNN-sup-nap in den binären Vektor pLH7000

Das Plasmid pBNN-sup-nap wurde mit dem Restriktionsenzym *Not*I linearisiert. Die überhängenden Enden wurden mit Klenow-Polymerase aufgefüllt. Anschließend wurde mit dem Restriktionsenzym *Hind*III gespalten. Das die Suppressionskassette enthaltende DNA-Fragment wurde isoliert und in den *Sma*I*-Hind*III-doppelverdauten binären Vektor pLH7000 kloniert (Hausmann and Töpfer (1999) Entwicklung von Plasmid-Vektoren in: Vorträge für die Pflanzenzüchtung Heft 45, "BioEngineering für Rapssorten nach Maß, -Ergebnisse eines BMBF-Forschungsverbunds-,,, Hrsg. D. Brauer, G. Röbbelen und R. Töpfer, Seiten 155-171).

Das resultierende Plasmid pLH7000-sup-nap wurde zur Transformation von Raps eingesetzt. Transgene Pflanzen mit gegenüber Wildtyppflanzen verringertem Sinapingehalt konnten selektioniert und intakte Pflanzen regeneriert werden.

In ähnlicher Weise wie oben insbesondere für *B. napus* beschrieben wurden auch Suppressionsvektoren für die Übertragung auf *A. thaliana* konstruiert. Der Unterschied bestand lediglich darin, dass anstelle des SGT1-Fragments ein Fragment von AtSGT1 in antisense- sowie sense-Orientierung kloniert wird. AtSGT1 ist in Milkowski et al. (2000) FEBS Letters 486: 183-184 beschrieben; in der Gendatenbank findet sich die Sequenz des SGT-Gens aus *A. thaliana* unter der Accession-No. BAB02351. Das amplifizierte Fragment enthält den 5'-Teil der codierenden Region der AtSGT1-cDNA (259 Nucleotide, Beginn mit dem A des Startcodons).

(Die dargestellte Sequenz zeigt nur den klonierten Teil aus AtSGT1 ,auf die Darstellung der angrenzenden Primer-Sequenzen wurde hier verzichtet.)

Die Klonierungsstrategie ist in Abbildung 1 dargestellt. dsRNAI steht für double strand RNA interference, wie oben erläutert.

Es ist klar, daß neben den oben erwähnten Vektoren pBNN und pLH7000 jeder andere für die Erstellung der gewünschten Antisense- bzw. dsRNAI-Konstrukte geeignete bzw. für die Pflanzentransformation geeignete Vektor eingesetzt werden kann. Entsprechende Vektoren sind dem auf dem Gebiet der Pflanzenbiotechnologie tätigen Fachmann bestens bekannt.

### Abbildungen

Abb. 1 zeigt in schematisierter Form die Erzeugung der dsRNAI-Suppressionskonstrukte.

## Patentansprüche

1. DNA-Sequenz, die ein Protein mit der enzymatischen Aktivität einer UDP-Glucose:Sinapinsäure-Glucosyltransferase (SGT) oder ein Fragment davon, das für die Inhibierung der endogenen SGT-Expression ausreicht, kodiert, ausgewählt aus der Gruppe, bestehend aus:
a) DNA-Sequenzen, die eine Nukleotidsequenz umfassen, die die in SEQ ID NO. 3 angegebene Aminosäuresequenz oder Fragmente davon kodiert,
b) DNA-Sequenzen, die die in SEQ ID No. 1 angegebene Nukleotidsequenz oder Teile davon umfassen, und
c) DNA-Sequenzen, die eine Nukleotidsequenz, die mit einem komplementären Strang der Nukleotidsequenz von a) oder b) hybridisieren, oder Teile dieser Nukleotidsequenz umfassen, und zu der in SEQ ID No. 1 angegebenen Nukleotidsequenz zu mindestens 60% identisch sind.

2. Rekombinantes Nukleinsäuremolekül, umfassend
a) regulatorische Sequenzen eines in Pflanzenzellen aktiven Promotors;
b) operativ damit verknüpft eine DNA-Sequenz nach Anspruch 1;
c) optional operativ damit verknüpft regulatorische Sequenzen, die als Transkriptions-, Terminations- und/oder Polyadenylierungssignale in Pflanzenzellen dienen können.

3. Rekombinantes Nukleinsäuremolekül nach einem der Ansprüche 1 oder 2, in dem die DNA-Sequenz ausschließlich oder zusätzlich zur sense-Orientierung in der Antisense-Orientierung vorliegt.

4. Rekombinantes Nukleinsäuremolekül nach einem der Ansprüche 1 oder 2, in dem die DNA-Sequenz als Cosuppressions-Konstrukt oder double strand RNA interference Suppressionskonstrukt vorliegt.

5. Rekombinantes Nukleinsäuremolekül nach einem der vorangehenden Ansprüche, worin der Promotor ein in Pflanzensamen aktiver Promotor, insbesondere ein USP-, LeB4-oder Napin-Promotor ist.

6. Vektor, umfassend ein rekombinantes Nukleinsäuremolekül nach einem der Ansprüche 1 bis 5.

7. Mikroorganismus, enthaltend ein rekombinantes Nukleinsäuremolekül oder einen Vektor nach einem der Ansprüche 1 bis 6.

8. Rekombinantes Protein mit der enzymatischen Aktivität einer UDP-Glucose:Sinapinsäure-Glucosyltransferase (SGT), kodiert von einer DNA-Sequenz nach Anspruch 1.

9. Verfahren zur Erzeugung von Pflanzen bzw. Pflanzenzellen mit einem gegenüber Wiltyppflanzen bzw. -pflanzenzellen verringerten Sinapingehalt, umfassend die folgenden Schritte:
a) Herstellung eines rekombinanten Nukleinsäuremoleküls, das folgende Sequenzen umfasst:
- regulatorische Sequenzen eines in Pflanzenzellen aktiven Promotors;
- operativ daran gebunden eine DNA-Sequenz nach Anspruch 1, die ein Protein mit der enzymatischen Aktivität einer SGT kodiert; und
- optional operativ daran gebunden regulatorische Sequenzen, die als Transkriptions-Terminations- und/oder Polyadenylierungssignale in Pflanzenzellen dienen können;
b) Übertragung des Nukleinsäuremoleküls aus a) auf pflanzliche Zellen; und
c) gegebenenfalls die Regeneration von Pflanzen aus den transformierten Pflanzenzellen.

10. Transgene Pflanzenzellen, enthaltend ein rekombinantes Nukleinsäuremolekül oder einen Vektor nach einem der Ansprüche 1 bis 6, oder hergestellt nach einem Verfahren nach Anspruch 9.

11. Transgene Pflanzen, enthaltend eine Pflanzenzelle nach Anspruch 10 oder hergestellt nach Anspruch 9, sowie Teile dieser Pflanzen, transgene Ernteprodukte und transgenes Vermehrungsmaterial dieser Pflanzen, wie Protoplasten, Pflanzenzellen, Kalli, Samen, Knollen, Stecklinge, sowie die transgenen Nachkommen dieser Pflanzen.

12. Transgene Pflanzen nach Anspruch 11, wobei es sich um Ölsaaten, insbesondere Raps handelt.

13. Verfahren zur Gewinnung von pflanzlichem Protein mit einem gegenüber Wildtyp-Pflanzenprotein verringerten Sinapingehalt, umfassend die folgenden Schritte:
a) die Übertragung eines rekombinanten Nukleinsäuremoleküls oder Vektors nach einem der Ansprüche 1 bis 6 auf Pflanzenzellen;
b) die Regeneration von Pflanzen aus den transformierten Pflanzenzellen;
c) die Verarbeitung der Samen der Pflanzen aus b) zur Gewinnung von pflanzlichem Protein mit einem verringertem Gehalt an Sinapin.

14. Verwendung einer DNA-Sequenz nach Anspruch 1 zur Verringerung des Sinapingehalts in Pflanzen, insbesondere Rapspflanzen.

15. Verwendung nach Anspruch 14, wobei die DNA-Sequenz in Antisense-Orientierung, als Cosuppressionskonstrukt oder als double strand RNA interference Suppressionskonstrukt verwendet wird.

16. Verwendung einer DNA-Sequenz nach Anspruch 1 in Antisense-Orientierung und/oder sense-Orientierung zur Erhöhung des Lecithingehalts in Pflanzen.

17. Verwendung einer DNA-Sequenz nach Anspruch 1 in Antisense-Orientierung und/oder sense-Orientierung zur Erhöhung des Resveratrolgehalts in Pflanzen.

18. Verwendung der nach Anspruch 9 hergestellten Pflanzen zur Gewinnung von pflanzlichem Protein mit einem im Vergleich zu Wildtyp-Pflanzenprotein verringerten Gehalt an Sinapin.

## Claims

1. A DNA sequence encoding a protein having the enzymatic activity of a UDP-glucose: sinapate glucosyl transferase (SGT), or a fragment thereof sufficient to inhibit the endogenous expression of SGT, selected from the group consisting of:
a) DNA sequences comprising a nucleotide sequence encoding the amino acid sequence given in SEQ ID No. 3 or fragments thereof,
b) DNA sequences comprising the nucleotide sequence given in SEQ ID No. 1 or parts thereof, and
c) DNA sequences comprising a nucleotide sequence hybridising with a complementary strand of the nucleotide sequence from a) or b) or parts of this nucleotide sequence, and with at least 60 % identity to the nucleotide sequence given in SEQ ID No. 1.

2. A recombinant nucleic acid molecule, comprising
a) regulatory sequences of a promoter that is active in plant cells;
b) operatively linked thereto a DNA sequence according to claim 1;
c) optionally, operatively linked thereto regulatory sequences that may serve as transcription, termination and/or polyadenylation signals in plant cells.

3. A recombinant nucleic acid molecule according to any one of claims 1 or 2, wherein the DNA sequence is present exclusively in the anti-sense orientation, or in addition to the sense orientation in the anti-sense orientation.

4. A recombinant nucleic acid molecule according to any one of claims 1 or 2, wherein the DNA sequence is present as co-suppression construct or as double strand RNA interference suppression construct.

5. A recombinant nucleic acid molecule according to any one of the preceding claims, wherein the promoter is a promoter that is active in plant seeds, in particular a USP, LeB4 or napin promoter.

6. A vector comprising a recombinant nucleic acid molecule according to any one of claims 1 to 5.

7. A micro-organism, containing a recombinant nucleic acid molecule or vector according to any one of claims 1 to 6.

8. A recombinant protein with the enzymatic activity of a UDP-glucose: sinapate glucosyl transferase (SGT), encoded by a DNA sequence according to claim 1.

9. A method for generating plants or plant cells having a reduced content of sinapine in comparison with wild-type plants or plant cells, comprising the following steps:
a) production of a recombinant nucleic acid molecule comprising the following sequences:
- regulatory sequences of a promoter that is active in plant cells;
- operatively linked thereto a DNA sequence according to claim 1, which codes for a protein having the enzymatic activity of an SGT; and
- optionally, operatively linked thereto regulatory sequences which may serve as transcription, termination and/or polyadenylation signals in plant cells;
b) transfer of the nucleic acid molecule from a) to plant cells; and
c) optionally, regeneration of plants from the transformed plant cells.

10. Transgenic plant cells, containing a recombinant nucleic acid molecule or a vector according to any one of claims 1 to 6, or produced by a method according to claim 9.

11. Transgenic plants, containing a plant cell according to claim 10 or produced according to claim 9, and parts of these plants, transgenic agricultural products and transgenic propagating material of these plants, such as protoplasts, plant cells, calli, seeds, tubers, cuttings and the transgenic progeny of these plants.

12. The transgenic plants according to claim 11, which are oil seeds, particularly rape.

13. A method for obtaining plant protein having a reduced content of sinapine compared to wild-type plant protein, comprising the following steps:
a) transfer of a recombinant nucleic acid molecule or a vector according to any one of claims 1 to 6 to plant cells;
b) regeneration of plants from the transformed plant cells;
c) processing of the seeds of the plants from b) for obtaining plant protein having a reduced content of sinapine.

14. Use of a DNA sequence according to claim 1 for the reduction of the sinapine content in plants, particularly in rapeseed plants.

15. Use according to claim 14, wherein the DNA sequence is used in anti-sense orientation as co-suppression construct or as double strand RNA interference suppression construct.

16. Use of a DNA sequence according to claim 1 in anti-sense orientation and/or in sense orientation to increase the content of lecithin in plants.

17. Use of a DNA sequence according to claim 1 in anti-sense orientation and/or in sense orientation to increase the content of resveratrol in plants.

18. Use of the plants produced according to claim 9 for obtaining plant protein having a reduced content of sinapine in comparison with wild-type plant protein.

## Revendications

1. Séquence d'ADN, qui code pour une protéine possédant l'activité enzymatique d'une UDP-glucose:acide sinapinique glucosyltransférase (SGT), ou pour un fragment de cette dernière, suffisant pour l'inhibition de l'expression endogène de la SGT, et qui est choisie dans le groupe constitué de :
a) séquences ADN, comprenant une séquence nucléotidique, codante pour la séquence d'acides aminés mentionnée sous SEQ ID NO. 3 ou pour des fragments de cette dernière,
b) séquences ADN, comprenant la séquence nucléotidique mentionnée dans SEQ ID No. 1 ou des parties de cette dernière,
c) séquences ADN, qui comprennent une séquence nucléotidique qui réalise une hybridation avec un brin complémentaire de la séquence nucléotidique de a) ou b), ou des parties de cette séquence nucléotidique, et qui sont à au moins 60% identiques à la séquence nucléotidique mentionnée sous SEQ ID No.l.

2. Molécule d'acide nucléique recombinante comprenant
a) des séquences régulatrices d'un promoteur actif dans des cellules végétales ;
b) combinées à ces dernières de façon opérationnelle, une séquence ADN selon la revendication 1 ;
c) optionnellement, combinées auxdites séquences de façon opérationnelle, des séquences régulatrices qui peuvent servir de signaux de transcription, de terminaison et/ou de polyadénylation dans des cellules végétales.

3. Molécule d'acide nucléique recombinante selon l'une des revendications 1 ou 2, dans laquelle la séquence ADN est présente, exclusivement ou en plus de l'orientation sens, dans l'orientation antisens.

4. Molécule d'acide nucléique recombinante selon l'une des revendications 1 ou 2, dans laquelle la séquence ADN est présente en tant que construction de cosuppression ou en tant que construction de suppression par interférence par ARN à double brin.

5. Molécule d'acide nucléique recombinante selon l'une des revendications précédentes, dans laquelle le promoteur est un promoteur actif dans des graines végétales, en particulier un promoteur USP, LeB4 ou de la napine.

6. Vecteur comprenant une molécule d'acide nucléique recombinante selon l'une des revendications 1 à 5.

7. Microorganisme contenant une molécule d'acide nucléique recombinante ou un vecteur selon l'une des revendications 1 à 6.

8. Protéine recombinante possédant l'activité enzymatique d'une UDP-glucose:acide sinapinique glucosyltransférase (SGT), codée par une séquence ADN selon la revendication 1.

9. Procédé pour la production de plantes, respectivement de cellules végétales possédant une teneur en sinapine réduite par rapport à des plantes, respectivement des cellules végétales de type sauvage, comportant les étapes suivantes :
a) préparation d'une molécule d'acide nucléique recombinante, qui comprend les séquences suivantes:
- des séquences régulatrices d'un promoteur actif dans des cellules végétales;
- combinées à ces dernières de façon opérationnelle, une séquence ADN selon la revendication 1, qui code pour une protéine possédant l'activité enzymatique d'une SGT; et
- optionnellement, combinées auxdites séquences de façon opérationnelle, des séquences régulatrices qui peuvent servir de signaux de transcription, de terminaison et/ou de polyadénylation dans des cellules végétales;
b) transfert de la molécule d'acide nucléique issue de a) sur des cellules végétales ; et
c) éventuellement la régénération de plantes à partir des cellules végétales transformées.

10. Cellules végétales transgéniques, contenant une molécule d'acide nucléique recombinante ou un vecteur selon l'une des revendications 1 à 6, ou préparées selon un procédé selon la revendication 9.

11. Plantes transgéniques contenant une cellule végétale selon la revendication 10 ou préparées selon la revendication 9, ainsi que des parties de ces plantes, des produits transgéniques de récolte et du matériau transgénique de reproduction de ces plantes, tels que des protoplastes, des cellules végétales, des cals, des semences, des tubercules, des boutures, ainsi que les descendants transgéniques de ces plantes.

12. Plantes transgéniques selon la revendication 11, dans lesquelles il s'agit de plantes oléifères, en particulier de colza.

13. Procédé pour l'obtention de protéine végétale ayant une teneur en sinapine réduite par rapport à la protéine végétale de type sauvage, comportant les étapes suivantes :
a) le transfert sur des cellules végétales d'une molécule d'acide nucléique recombinante ou d'un vecteur selon l'une des revendications 1 à 6;
b) la régénération de plantes à partir des cellules végétales transformées ;
c) le traitement des semences des plantes issus de b) en vue de l'obtention de protéine végétale ayant une teneur réduite en sinapine.

14. Utilisation d'une séquence ADN selon la revendication 1 en vue de limiter la teneur en sinapine dans des plantes, en particulier des plants de colza.

15. Utilisation selon la revendication 14, dans laquelle la séquence ADN est utilisée en orientation antisens, en tant que construction de cosuppression ou en tant que construction de suppression par interférence par ARN à double brin.

16. Utilisation d'une séquence ADN selon la revendication 1 en orientation antisens et/ou en orientation sens, en vue de l'augmentation de la teneur en lécithine dans des plantes.

17. Utilisation d'une séquence ADN selon la revendication 1 en orientation antisens et/ou en orientation sens, en vue de l'augmentation de la teneur en resvératrol dans des plantes.

18. Utilisation des végétaux préparés selon la revendication 9, en vue de l'obtention de protéine végétale ayant une teneur en sinapine réduite par rapport à une protéine végétale de type sauvage.


### SEQUENZPROTOKOLL

(1) ALLGEMEINE ANGABEN:
   (i) ANMELDER :
      (A) NAME: Institut fuer Pflanzenbiochemie Halle
      (B) STRASSE: Weinberg 3
      (C) ORT: Halle
      (E) LAND: Deutschland
      (F) POSTLEITZAHL: 06120
   (ii) BEZEICHNUNG DER ERFINDUNG: Verfahren zur Beeinflussung des Sinapingehalts in transgenen Pflanzen und Pflanzenzellen
   (iii) ANZAHL DER SEQUENZEN: 3
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: Patent In Release #1.0, Version #1.30 (EPA)
(2) ANGABEN ZU SEQ ID NO: 1:
   (i) SEQUENZKENNZEICHEN:
      (A) LÄNGE: 1715 Basenpaare
      (B) ART: Nucleotid
      (C) STRANGFORM: Doppelstrang
      (D) TOPOLOGIE: linear
   (ii) ART DES MOLEKÜLS: cDNA
   (xi) SEQUENZBESCHREIBUNG: SEQ ID NO: 1:
   GAAAAACAGA GCACACAGAA GAGAACCCCT TTTTGAAGGA TGGAACTATC ATCTTCTCCT   60
   TTACCTCCTC ATGTTATGCT TGTATCCTTC CCAGGACAAG GCCACGTTAA TCCACTTCTT  120
   CGTCTCGGCA AGCTCTTAGC TTCGAAGGGT TTACTCGTCA CTTTTGTCAC CACAGAATCA  180
   TGGGGCAAAA AGATGCGAAC CGCCAACAAG ATTCAAGACC GAGCCCTCAA ACCTATCGGT  240
   AAAGGTTATC TCCGGTTCGA TTTCTTCAAC GATGGGCTCC CTGAAGACGA CGATGCAAGC  300
   AGAACCAACT TAACCATCCT CCGACCACAA CTCGAGCTGG TCGGACAACA AGAGATCAAA  360
   AACCTCGTGA AACGTTACAA GGAAGTGATG AAACAGCCCG TGACGTGTCT CATCAACAAC  420
   CCTTTCGTCT CTTGGGTCTG TGACGTAGCC GAAGATCTTC AAATCCCCTG TGCTGTTCTC  480
   TGGGTCCAGT CTTGTGCTTG CCTAGCTTCT TATTATTATT ACAACCACAA GCTTGTCGAC  540
   TTCCCGACCG AAACAGATCC CAAGATCGAT GTCCAGATCC CGTGCATGCC TGTCTTGAAA  600
   CACGACGAGA TCCCTTCTTT CATCCATCCT TTTTCACCTT ATTCGGGTTT AAGAGAAGTG  660
   ATCATTGATC AGATCAAACG TCTTCACAAG CCTTTCGCTG TTCTCATCGA TACTTTCTAC  720
   TCCTTGGAGA AAGATATCAT CGACCACATG ACAAACCTCT CTCGCACCGG CTTTGTCAGA  780
   CCGCTCGGAC CGCTTTACAA AATGGCCAAA ACGTTGATTT GTGATGACAT CAAAGGAGAT  840
   ATGTCTGAGA CGAGGGATGA CTGCATGGAG TGGTTAGACT CGCAGCCTGT TTCGTCCGTT 900
   GTTTACATCT CATTTGGTAC CGTGGCTTAC GTGACACAAG AACAGATCAG CGAGATTGCG 960
   TTAGGCGTTT TAAACGCTGA CGTTTCGTTC TTGTGGGTGA TAAGACAACA AGAACTAGGT 1020
   GTAAACAAAG AGCGACATGT TCTGCCTGAA GAACTCAAAG GGAAAGGTAA AGTCATTGAA 1080
   TGGTGTTCAC AAGAGAAAGT CTTAGCTCAT CCTTCTGTGG TTTGTTTCGT GACTCATTGT 1140
   GGATGGAACT CAACGATGGA AGCTTTGTCT AGTGGAGTCC CAACGGTCTG TTTTCCTCAG 1200
   TGGGGAGATC AAGTCACCGA CGCTGCTTAC ATGATCGACG TGTTCAAGAC GGGAGTGAGG 1260
   CTTAGCCGTG GAGAGACGGA GGAGAGGGTG GTGCCTAGGG AGGAAGTAGC GGAGAGGCTG 1320
   AGAGAAGTTA CGAAAGGAGA GAAAGCGACG GAGCTGAAGA AGAATGCTTT AAAATGGAAG 1380
   GAGGAGGCGG AAGCGGCCGT GGCTCGCGGT GGTTCGTCGG ATCGGAATCT TGATGAGTTT 1440
   GTGGAAAAGT TGGGCGTGAA ACCTGTGGCT AAACAGAACG GAAGTCTCAA TCAAAACGGA 1500
   AGTATTCAAA AACTTTTATT GCAAAAGTCA TAAATGTGTT TTGTATTATC TGTTGTTTGT 1560
   TTTCTGCAAA TCCTGATAAT TGTGTTTGTT TGTATGCTCC CTCCGTTCTT TGTTCTCGTT 1620
   TTTTTTTTGT ATGGGTTTGG GGAGACTAGT TATCCTTCTT GAATAAATGT TAGATCACGA 1680
   CTTGTGTTCT TAAGATTAAA AAAAAAAAAA AAAAA 1715
(2) ANGABEN ZU SEQ ID NO: 2:
   (i) SEQUENZKENNZEICHEN:
      (A) LÄNGE: 1715 Basenpaare
      (B) ART: Nucleotid
      (C) STRANGFORM: Doppelstrang
      (D) TOPOLOGIE: linear
   (ii) ART DES MOLEKÜLS: cDNA
   (ix) MERKMAL:
      (A) NAME/SCHLÜSSEL: CDS
      (B) LAGE:40..1533
   (xi). SEQUENZBESCHREIBUNG: SEQ ID NO: 2:
(2) ANGABEN ZU SEQ ID NO: 3:
   (i) SEQUENZKENNZEICHEN:
      (A) LÄNGE: 498 Aminosäuren
      (B) ART: Aminosäure
      (D) TOPOLOGIE: linear
   (ii) ART DES MOLEKÜLS: Protein
   (xi) SEQUENZBESCHREIBUNG: SEQ ID NO: 3:
   Met Glu Leu Ser Ser Ser Pro Leu Pro Pro His Val Met Leu Val Ser
   1 5 10 15
   Phe Pro Gly Gln Gly His Val Asn Pro Leu Leu Arg Leu Gly Lys Leu
   20 25 30
   Leu Ala Ser Lys Gly Leu Leu Val Thr Phe Val Thr Thr Glu Ser Trp
   35 40 45
   Gly Lys Lys Met Arg Thr Ala Asn Lys Ile Gln Asp Arg Ala Leu Lys
   50 55 60
   Pro Ile Gly Lys Gly Tyr Leu Arg Phe Asp Phe Phe Asn Asp Gly Leu
   65 70 75 80
   Pro Glu Asp Asp Asp Ala Ser Arg Thr Asn Leu Thr Ile Leu Arg Pro
   85 90 95
   Gln Leu Glu Leu Val Gly Gln Gln Glu Ile Lys Asn Leu Val Lys Arg
   100 105 110
   Tyr Lys Glu Val Met Lys Gln Pro Val Thr Cys Leu Ile Asn Asn Pro
   115 120 125
   Phe Val Ser Trp Val Cys Asp Val Ala Glu Asp Leu Gln Ile Pro Cys
   130 135 140
   Ala Val Leu Trp Val Gln Ser Cys Ala Cys Leu Ala Ser Tyr Tyr Tyr
   145 150 155 160
   Tyr Asn His Lys Leu Val Asp Phe Pro Thr Glu Thr Asp Pro Lys Ile
   165 170 175
   Asp Val Gln Ile Pro Cys Met Pro Val Leu Lys His Asp Glu Ile Pro
   180 185 190
   Ser Phe Ile His Pro Phe Ser Pro Tyr Ser Gly Leu Arg Glu Val Ile
   195 200 205
   Ile Asp Gln Ile Lys Arg Leu His Lys Pro Phe Ala Val Leu Ile Asp
   210 215 220
   Thr Phe Tyr Ser Leu Glu Lys Asp Ile Ile Asp His Met Thr Asn Leu
   225 230 235 240
   Ser Arg Thr Gly Phe Val Arg Pro Leu Gly Pro Leu Tyr Lys Met Ala
   245 250 255
   Lys Thr Leu Ile Cys Asp Asp Ile Lys Gly Asp Met Ser Glu Thr Arg
   260 265 270
   Asp Asp Cys Met Glu Trp Leu Asp Ser Gln Pro Val Ser Ser Val Val
           275                     280                     285
   Tyr Ile Ser Phe Gly Thr Val Ala Tyr Val Thr Gln Glu Gln Ile Ser
       290                     295                     300
   Glu Ile Ala Leu Gly Val Leu Asn Ala Asp Val Ser Phe Leu Trp Val
   305                     310                     315                     320
   Ile Arg Gln Gln Glu Leu Gly Val Asn Lys Glu Arg His Val Leu Pro
                   325                     330                     335
   Glu Glu Leu Lys Gly Lys Gly Lys Val Ile Glu Trp Cys Ser Gln Glu
               340                     345                     350
   Lys Val Leu Ala His Pro Ser Val Val Cys Phe Val Thr His Cys Gly
           355                     360                     365
   Trp Asn Ser Thr Met Glu Ala Leu Ser Ser Gly Val Pro Thr Val Cys
       370                     375                     380
   Phe Pro Gln Trp Gly Asp Gln Val Thr Asp Ala Ala Tyr Met Ile Asp
   385                     390                     395                     400
   Val Phe Lys Thr Gly Val Arg Leu Ser Arg Gly Glu Thr Glu Glu Arg
                   405                     410                     415
   Val Val Pro Arg Glu Glu Val Ala Glu Arg Leu Arg Glu Val Thr Lys
               420                     425                     430
   Gly Glu Lys Ala Thr Glu Leu Lys Lys Asn Ala Leu Lys Trp Lys Glu
           435                     440                     445
   Glu Ala Glu Ala Ala Val Ala Arg Gly Gly Ser Ser Asp Arg Asn Leu
       450                     455                     460
   Asp Glu Phe Val Glu Lys Leu Gly Val Lys Pro Val Ala Lys Gln Asn
   465                     470                     475                     480
   Gly Ser Leu Asn Gln Asn Gly Ser Ile Gln Lys Leu Leu Leu Gln Lys
                   485                     490                     495
   Ser  *